(19) Europäisches Patentamt
European Patent Office
Office européen des brevets

(11) Veröffentlichungsnummer: **0 007 428**
**A1**

(12) # EUROPÄISCHE PATENTANMELDUNG

(21) Anmeldenummer: 79101988.8

(22) Anmeldetag: 18.06.79

(51) Int. Cl.³: C 07 D 409/12
C 07 D 405/12, A 61 K 31/415

(30) Priorität: 18.07.78 DE 2831480

(43) Veröffentlichungstag der Anmeldung:
06.02.80 Patentblatt 80/3

(84) Benannte Vertragsstaaten:
AT BE CH DE FR GB IT LU NL SE

(71) Anmelder: C.H. BOEHRINGER SOHN
Postfach 200
D-6507 Ingelheim am Rhein(DE)

(72) Erfinder: Stähle, Helmut, Dr.
Rotweinstrasse 23
D-6507 Ingelheim(DE)

(72) Erfinder: Köppe, Herbert, Dr.
Neuweg 72
D-6507 Ingelheim(DE)

(72) Erfinder: Kummer, Werner, Dr.
Georg-Scheuing-Strasse 15
D-6507 Ingelheim(DE)

(72) Erfinder: Stockhaus, Klaus, Dr.
Pfarrer-Heberer-Strasse 35
D-6530 Bingen(DE)

(72) Erfinder: Gaida, Wolfram, Dr.
Bahnhofstrasse 74
D-6507 Ingelheim(DE)

(72) Erfinder: Pichler, Ludwig, Dr.
Gusshausstrasse 24
A-1040 Wien(AT)

(54) **Neue substituierte 2-Phenylamino-imidazoline-(2), deren Säureadditionssalze, diese enthaltende Arzneimittel und Verfahren zur Herstellung derselben.**

(57) Die Erfindung betrifft substituierte 2- Phenylamino-imidazoline der allgemeinen Formel I

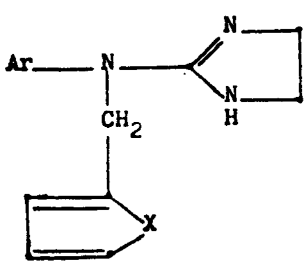

I

worin Ar 2,6-Dibromphenyl, 2,4-Dibromphenyl oder 3-Brom-4-fluorphenyl und X Sauerstoff oder Schwefel bedeuten sowie deren Säureadditionssalze.

Die neuen Verbindungen sind als Herz- bzw. Coronartherapeutika verwendbar.

EP 0 007 428 A1

Croydon Printing Company Ltd.

Die Erfindung betrifft neue substituierte 2-Phenylamino-imidazoline-(2) der allgemeinen Formel

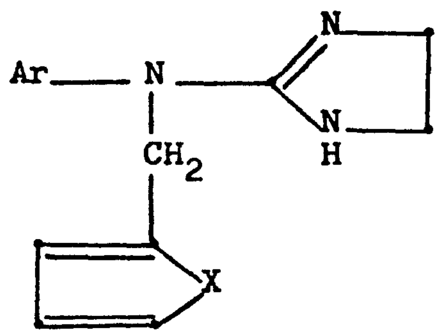

I

sowie deren physiologisch verträgliche Säureadditionssalze mit wertvollen therapeutischen Eigenschaften.

In der Formel I bedeutet Ar 2,6-Dibromphenyl, 2,4-Dibromphenyl oder 3-Brom-4-fluorphenyl.

X bedeutet Sauerstoff oder Schwefel.

Die Herstellung der Verbindungen der Formel I erfolgt durch:

a) Umsetzung eines 2-Phenylimino-imidazolidins der allgemeinen Formel

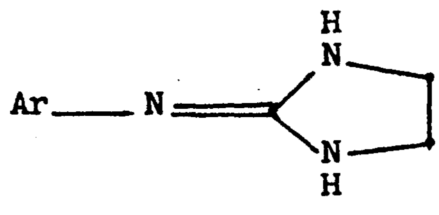

II

in der Ar die oben genannte Bedeutung besitzt, mit einem Halogenid der allgemeinen Formel

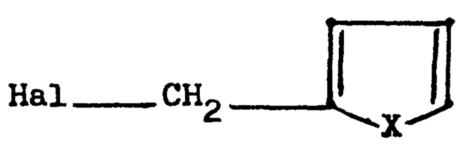

III

worin Hal ein Chlor-, Brom- oder Jodatom bedeutet und X die obige Bedeutung hat; oder

b) Umsetzung einer Verbindung der allgemeinen Formel

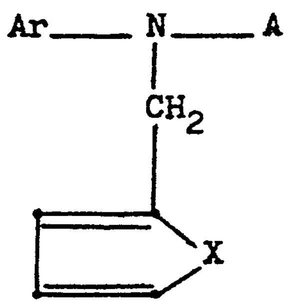

IV

in der Ar und X wie oben angegeben definiert sind und A eine Cyanogruppe oder den Rest 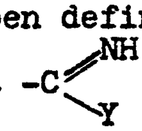 bedeutet, wobei Y eine Alkoxy- oder Alkylthiogruppe mit bis zu 4 C-Atomen oder eine Sulfhydryl- oder Aminogruppe darstellt, mit Äthylendiamin oder dessen Säureadditionssalzen.

Bei der Alkylierung der 2-Arylimino-imidazolidine der Formel II nach Verfahren a) erfolgt die Substitution ausschließlich am Brückenstickstoffatom. Bei der Umsetzung nach den Verfahren b) ist die Konstitution der Endverbindungen durch die Synthese festgelegt. Die Position der Substituenten läßt sich außer durch die Synthese auch durch die NMR-Spektroskopie festlegen. (Vgl. H. Stähle und K.-H. Pook, Liebigs Ann. Chem. 751, 159 ff (1971).

Die Umsetzung nach Verfahren a) erfolgt zweckmäßigerweise durch Erhitzen der Reaktionspartner - vorzugsweise in Gegenwart eines polaren oder unpolaren organischen Lösungsmittels - auf Temperaturen von etwa 50 bis 150°C. Die speziellen Reaktionsbedingungen hängen in starkem Maße von der Reaktivität der Umsetzungsteilnehmer ab. Es empfiehlt sich, bei der

Alkylierung das Halogenid im Überschuß anzuwenden und die Umsetzung in Gegenwart eines säurebindenden Mittels durchzuführen.

Bei dem Verfahren nach b) ist es erforderlich, bei erhöhter Temperatur, zwischen 60 °C und 180 °C zu arbeiten. Lösungsmittel sind nicht erforderlich. Es ist zweckmäßig, das als Reaktionspartner verwendete Äthylendiamin bzw. dessen Säureadditionssalz, im Überschuß anzuwenden.

Ausgangsverbindungen der Formel II sind z. B. in den belgischen Patenten Nr. 623 305, 687 657 und 705 944 beschrieben.

Ausgangsverbindungen der Formel III lassen sich durch Halogenierung der zugrundeliegenden primären Alkohole darstellen.

Verbindungen der Formel IV erhält man ausgehend von Anilinen, durch Umsetzung mit Verbindungen der Formel III und nachfolgende Reaktion der dabei entstehenden sekundären Amine, mit Cyanaten oder Thiocyanaten wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. $H_2S$-Abspaltung aus Thioharnstoffen mittels Blei- oder Quecksilbersalzen, gelangt man zu Cyanamiden, an die Ammoniak unter Bildung von Guanidinen angelagert werden kann.

Die erfindungsgemäßen 2-Phenylamino-imidazoline-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen geeignet. Die Beeinflussung der Herzfrequenz wurde an Kaninchen und an Spinalratten sowie an intakten, narkotisierten Ratten untersucht.
Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

Beispiel 1

2-[N-(2,6-Dibromphenyl)-N-(2-thienylmethyl)amino]-2-imidazolin-hydrochlorid

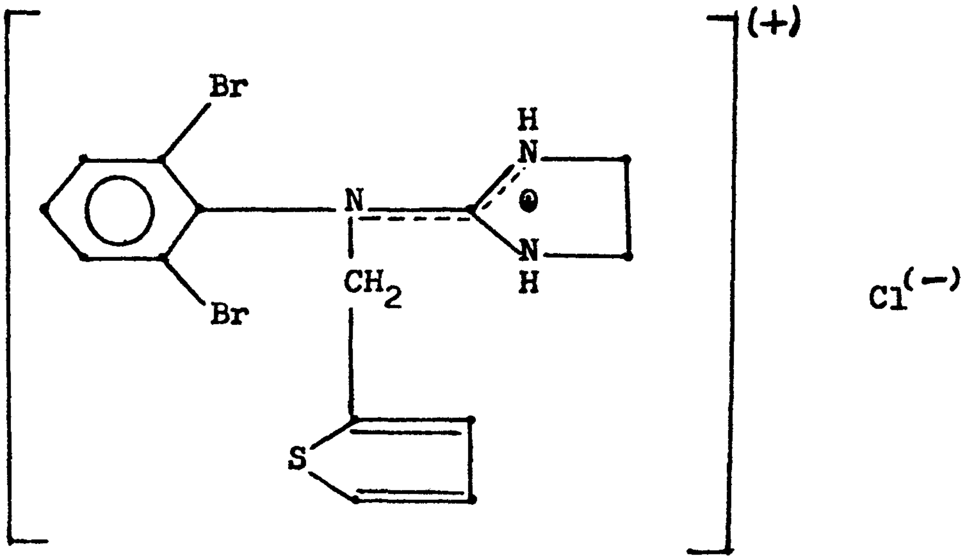

63,8 g (0,2 Mol) 2-(2,6-Dibromphenylimino)imidazolidin werden zusammen mit 33,1 g (125 %) 2-Chlormethyl-thiophen und 30 ml Triäthylamin in 400 ml absolutem Toluol 6 Stunden lang unter Rühren am Rückfluß erhitzt. Nach dieser Zeit wird abgesaugt und der aus dem neuen Imidazolin-derivat bestehende Filterrückstand mehrmals mit Toluol gewaschen. Sodann löst man ihn in etwa 3 Liter Wasser unter Zugabe von 25 ml 2 N HCl. Die salzsaure Lösung wird zweimal mit je 300 ml Äther extrahiert (Ätherextrakte werden verworfen) und durch Zugabe von 5 N NaOH auf pH 7 eingestellt. Nach erneutem Extrahieren der neutralen Lösung mit 300 ml Äther (Ätherextrakt wird verworfen) wird bei aufsteigenden pH-Werten (stufenweises Alkalisieren mit 2 N NaOH; jeweils etwa 3 ml) noch solange fraktioniert mit Äther extrahiert, bis alle Verunreinigungen aus der wässrigen Phase entfernt sind (laufende Kontrolle durch Dünnschichtchromatogramm). Anschließend wird die wässrige Lösung mit 200 ml 2 N NaOH versetzt, wobei sich die Imidazolinbase in reiner Form abscheidet. Ausbeute nach Absaugen, Waschen mit Wasser und Trocknen: 48,4 g (58,3 % der Theorie). Schmelzpunkt: 106 - 108°C. Das Hydrochlorid schmilzt bei 239 - 240°C.

$C_{14}H_{13}Br_2N_3S \times HCl$ (451,62)

| | C | H | Br | Cl | N | S |
|---|---|---|---|---|---|---|
| Ber.: | 37,23 | 3,12 | 35,39 | 7,85 | 9,30 | 7,10 |
| Gef.: | 37,32 | 3,11 | 35,22 | 8,19 | 9,12 | 6,91 |

Dünnschichtchromatogramm:

Rf: 0,7; Fließmittelsystem: Benzol : Dioxan : Äthanol : konz. $NH_4OH$, Nachweis: Jodplatinat 50 : 40 : 5 : 5

Analog dem vorstehenden Beispiel wurden die folgenden Verbindungen hergestellt, Die Schmelzpunkte beziehen sich auf die Basen der Formel I.

| Beispiel Nr. | Ar | X | Fp °C | Ausbeute % der Theorie |
|---|---|---|---|---|
| 2 | 2,6-Dibromphenyl | O | 114-115 | 25,1 |
| 3 | 2,4-Dibromphenyl | O | 130-131 | 30,9 |
| 4 | 3-Brom-4-fluor-phenyl | O | 87-88 | 41,4 |
| 5 | 2,4-Dibromphenyl | S | 121-122 | 29,3 |

Formulierungsbeispiele

Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:
Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:
Wirkstoff und Natriumchlorid werden in Wasser gelöst und unter Stickstoff in Glasampullen abgefüllt.

Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**PATENTANSPRÜCHE:**

1. Substituierte 2-Phenylamino-imidazoline-(2) der allgemeinen Formel

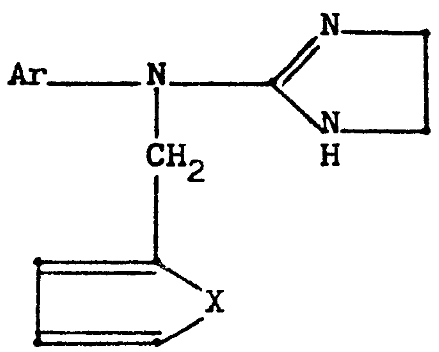

I

worin Ar 2,6-Dibromphenyl, 2,4-Dibromphenyl oder 3-Brom-4-fluorphenyl und X Sauerstoff oder Schwefel bedeuten sowie deren Säureadditionssalze.

2. Verfahren zur Herstellung von substituierten 2-Phenylaminoimidazolinen-(2) der allgemeinen Formel I nach Anspruch 1 und von deren Säureadditionssalzen, dadurch gekennzeichnet, daß man

a) ein 2-Phenylimino-imidazolidin der allgemeinen Formel

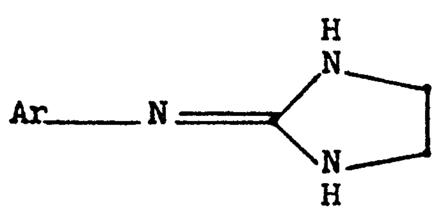

II

in der Ar die oben genannten Bedeutungen besitzt, mit einem Halogenid der allgemeinen Formel

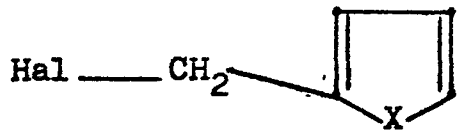 III

worin Hal ein Chlor-, Brom- oder Jodatom bedeutet und X die oben benannte Bedeutung hat, umsetzt oder

b) eine Verbindung der allgemeinen Formel

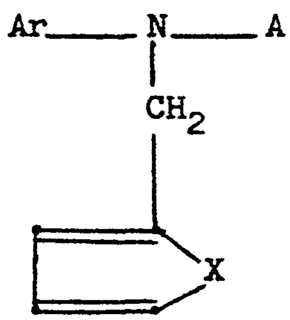 IV

in der Ar und X wie oben angegeben definiert sind und A eine Cyanogruppe oder den Rest 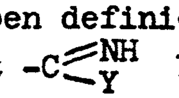 bedeutet, wobei Y eine Alkoxy- oder Alkylthiogruppe mit bis zu 4 Kohlenstoffatomen oder eine Sulfhydryl- oder Aminogruppe darstellt, mit Äthylendiamin oder dessen Säureadditionssalzen umsetzt, und daß man gegebenenfalls das nach einem der Verfahren erhaltene Endprodukt in ein Säureadditionssalze überführt.

3. Verfahren nach Anspruch 2, dadurch gekennzeichnet, daß man die Umsetzung bei Temperaturen von etwa 50 bis 100°C durchführt.

4. Verfahren nach Anspruch 2 a) und/oder 3, dadurch gekennzeichnet, daß man die Umsetzung in Gegenwart eines polaren oder unpolaren organischen Lösungsmittels durchführt.

5. Verfahren nach Anspruch 2 a) bis 4, dadurch gekennzeichnet, daß man die Umsetzung in Gegenwart eines säurebindenden Mittels durchführt.

6. Pharmazeutische Zubereitungen, dadurch gekennzeichnet, daß sie als Wirkstoff eine oder mehrere Verbindungen der allgemeinen Formel I oder deren Säureadditionssalze enthalten.

7. Verfahren zur Herstellung von Arzneimitteln nach Anspruch 6, dadurch gekennzeichnet, daß man eine oder mehrere Verbindungen der allgemeinen Formel I oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

8. Verwendung von Verbindungen der allgemeinen Formel I oder deren physiologisch verträglichen Säureadditionssalzen bei der Bekämpfung von Herz- und Coronarerkrankungen.

Europäisches Patentamt

# EUROPÄISCHER RECHERCHENBERICHT

Nummer der Anmeldung

EP 79 101 988.8

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| X | DE - A - 2 308 883 (BOEHRINGER)<br>* Seite 14, Zeile 18, Ansprüche 1 bis 13 *<br>-- | 1-8 | C 07 D 409/12<br>C 07 D 405/12<br>A 61 K 31/415 |
| | DD - A - 99 797 (BOEHRINGER)<br>* Anspruch 1 *<br>-- | 2 | |
| | US - A - 3 850 926 (BOEHRINGER)<br>* Beispiel 12 *<br>---- | 2 | |

RECHERCHIERTE SACHGEBIETE (Int. Cl.3)

A 61 K 31/415
C 07 D 405/12
C 07 D 409/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 02-11-1979 | KAPTEYN |